# EUROPÄISCHE PATENTSCHRIFT

(11) **EP 1 549 650 B1**
(45) Veröffentlichungstag und Bekanntmachung des Hinweises auf die Patenterteilung: **22.03.2006**
(21) Anmeldenummer: 03807842.4
(22) Anmeldetag: 06.10.2003
(51) Int. Cl.: C07D 471/04, A61K 31/437, A61P 25/00, A61P 35/00, C07D 235/00, C07D 221/00

(54) **SUBSTITUIERTE C-IMIDAZO[1,2-A]LPYRIDIN-3YL-METHYLAMINE**
SUBSTITUTED C-IMIDAZO[1,2-A]PYRIDIN-3-YL-METHYLAMINES
C-IMIDAZO[1,2-A]PYRIDIN-3YL-METHYLAMINES SUBSTITUEES

(30) Priorität: 08.10.2002 DE 10246890
(43) Veröffentlichungstag der Anmeldung: 06.07.2005
(73) Patentinhaber: Grünenthal GmbH, 52078 Aachen (DE)
(72) Erfinder: HENNIES, Hagen-Heinrich, 52152 Simmerath (DE); SUNDERMANN, Corinna, 52066 Aachen (DE); SUNDERMANN, Bernd, 52066 Aachen (DE); OBERBÖRSCH, Stefan, 52074 Aachen (DE)
(86) Internationale Anmeldenummer: PCT/EP2003/011011
(87) Internationale Veröffentlichungsnummer: WO 2004/033453

(56) Entgegenhaltungen:
- WO-A-01/27109
- WO-A-02/14313
- WO-A-02/066477

## Beschreibung

Die vorliegende Erfindung betrifft substituierte C-Imidazo[1,2-a]pyridin-3-yl-methylamine sowie ihrer physiologisch verträgliche Salze, Verfahren zu deren Herstellung, Arzneimittel enthaltend diese Verbindungen und die Verwendung von substituierten C-Imidazo[1,2-a]pyridin-3-yl-methylaminen zur Herstellung von Arzneimitteln.

Stickstoffmonoxid (NO) reguliert zahlreiche physiologische Prozesse, unter anderem die Neurotransmission, die Relaxation und Proliferation von glatter Muskulatur, die Adhäsion und Aggregation von Thrombozyten sowie die Gewebeverletzung und Entzündung. Aufgrund der Vielzahl von Signalfunktionen wird Stickstoffmonoxid mit einer Reihe von Krankheiten in Verbindung gebracht, beispielsweise in L. J. lgnarro, Angew. Chem. (1999), 111, Seiten 2002-2013 und in F. Murad, Angew. Chem. lnt. Ed. (1999), 111, Seiten 1976-1989. Eine wichtige Rolle bei der therapeutischen Beeinflussung dieser Krankheiten spielt dabei das für die physiologische Bildung von Stickstoffmonoxid verantwortliche Enzym, die Stickstoffmonoxid-Synthase (NO-Synthase). Bislang wurden drei verschiedene lsoformen der NO-Synthase identifiziert, nämlich die beiden konstitutiven Formen nNO-Synthase und eNO-Synthase sowie die induzierbare Form iNO-Synthase (A. J. Hobbs, A. Higgs, S. Moncada, Annu. Rev. Pharmacol. Toxicol. (1999), 39, Seiten 191-220; I. C. Green, P.-E. Chabrier, DDT (1999), 4, Seiten 47.-49; P.-E. Chabrier et al., Cell. Mol. Life Sci. (1999), 55, Seiten 1029-1035).

Die Hemmung der NO-Synthase eröffnet neue Therapieansätze für verschiedene Krankheiten, die mit Stickstoffmonoxid in Zusammenhang stehen (A. J. Hobbs et al., Annu. Rev. Pharmacol. Toxicol. (1999), 39, Seiten 191-220; l. C. Green, P.-E. Chabrier, DDT (1999),4, Seiten 47-49; P.-E. Chabrier et al., Cell. Mol. Life Sci. (1999), 55, Seiten 1029-1035), wie beispielsweise Migräne (L. L. Thomsen, J. Olesen, Clinical Neuroscience (1998), 5, Seiten 28-33; L. H. Lassen et al., The Lancet (1997), 349, 401-402), septischer Schock, neurodegenerative Erkrankungen wie Multiple Sklerose, Morbus Parkinson, Morbus Alzheimer oder Morbus Huntington, Entzündungen, Entzündungsschmerz, cerebrale Ischämie, Diabetes, Meningitis und Arteriosklerose. Darüber hinaus kann die Inhibierung der NO-Synthase einen Effekt auf die Wundheilung, auf Tumoren und auf die Angiogenese haben sowie eine unspezifische Immunität gegen Mikroorganismen bewirken (A. J. Hobbs et al., Annu. Rev. Pharmacol. Toxicol. (1999), 39, Seiten 191-220).

Bislang bekannte Wirkstoffe, die die NO-Synthase hemmen, sind neben L-NMMA und L-NAME- d.h. Analoga des L-Arginins, aus dem in-vivo unter Beteiligung von NO-Synthase Stickstoffmonoxid und Citrullin gebildet werden - u.a. S-Methyl-L-citrullin, Aminoguanidin, S-Methylisoharnstoff, 7-Nitroindazol und 2-Mercaptoethylguanidin (A. J. Hobbs et al., Annu. Rev. Pharmacol. Toxicol. (1999), 39, Seiten 191-220).

WO 02/14313 offenbart substituierte Imidazo[1,2-a]pyridin-3-yl-Verbindungen, die inhibitorisch in den Prozess der β-Amyloid-Entstehung bzw. dessen Freisetzung aus Zellen eingreifen und sich daher insbesondere zur Therapie der Alzheimerschen Krankheit eignen. Bei diesen Verbindungen ist der Stickstoff, der nicht Bestandteil des heteroaromatischen Systems ist, immer in einen Heterocyclus eingebunden.

WO 01/27109 offenbart substituierte lmidazo[1,2-a]pyridin-3-yl-Verbindungen, die sich zur Herstellung eines Medikaments zur NO-Sythase-Inhibierung eignen. Bei diesen Verbindungen ist der exocyclische Stickstoff direkt an den aromatischen Heterocyclus gebunden.

WO 02/066477 offenbart substituierte Imidazo[1,2-a]pyridin-3-yl-Verbindungen, die das Gonadotropin-freisetzende Hormon antagonisieren. Die Verbindungen weisen spezielle Reste in den Positionen R¹ und R² am Imidazopyridin auf.

Eine Aufgabe der vorliegenden Erfindung bestand daher darin, neue Substanzen zur Verfügung zu stellen, die als Inhibitor auf die Stickstoffmonoxid-Synthase wirken. Insbesondere sollen sich die Arzneimittel zur Behandlung von Migräne, septischem Schock, neurodegenerativer Krankheiten, wie Multipler Sklerose, Morbus Parkinson, Morbus Alzheimer oder Morbus Huntington, Entzündungen, Entzündungsschmerz, cerebraler lschämie, Diabetes, Meningitis, Arteriosklerose, Krebserkrankungen, Pilzerkrankungen oder zur Wundheilung eignen.

Es wurde gefunden, daß substituierte C-Imidazo[1,2-a]pyridin-3-yl-methylamine der nachstehenden allgemeinen Formel 1 als Inhibitoren der Stickstoffmonoxid-Synthase wirken und sich insbesondere zur Behandlung von Migräne, septischem Schock, neurodegenerativen Erkrankungen, wie Multipler Sklerose, Morbus Parkinson, Morbus Alzheimer oder Morbus Huntington, Entzündungen, Entzündungsschmerz, cerebraler lschämie, Diabetes, Meningitis, Arteriosklerose, Krebserkrankungen, Pilzerkrankungen oder zur Wundheilung eignen.

Gegenstand der vorliegenden Erfindung sind daher substituierte C-Imidazo[1,2-a]pyridin-3-yl-methylamine der allgemeinen Formel I, 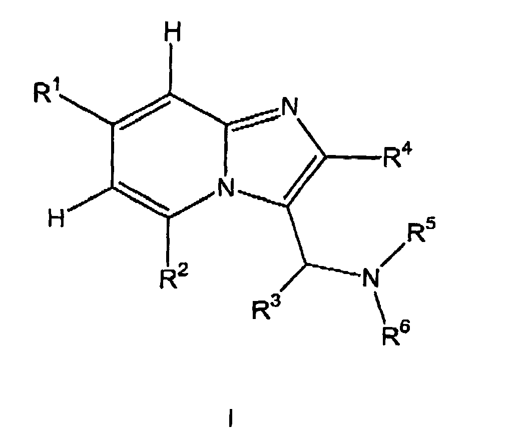 worin jeweils
- R¹: für OH, SH, NH₂, NHCH₃, N(CH₃)₂, CH₃, CH₂Cl, CH₂F, CHF₂, CF₃, OCH₃, OCH₂Cl, OCH₂F, OCHF₂, OCF₃, C₂H₅, CHClCH₃, CH₂CH₂Cl, CHFCH₃, CH₂CH₂F, CH₂CHF₂, CH₂CF₃, OC₂H₅, COOH, CH₂OH, CHOHCH₃ oder CH₂CH₂OH steht,
- R²: für H oder CH₃ steht,
- R³: für H steht,
- R⁴: für H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl, Hexyl, Heptyl, Octyl, Nonyl, Decyl, Undecyl und Dodecyl; jeweils unsubstituiert oder ein- oder mehrfach substituiert mit einem Substituenten ausgewählt aus der Gruppe bestehend aus F, Cl, Br, I, NH₂, SH und OH; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF₃, Methoxy und Ethoxy; oder einen Rest gemäß Formel II steht, 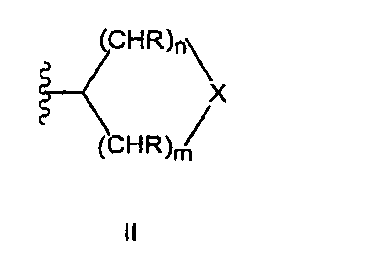
worin jeweils
- n: für eine Zahl zwischen 0 und 6 steht,
- m: für eine Zahl zwischen 0 und 6 steht,
1 ≤ m+n ≤ 6 ist,
- X: O, S, SO, SO₂ oder NR⁷ steht,
- R: unabhängig voneinander für H, F, Br, I, Cl, OH, SH, NH₂, NO₂, CH₃, C₂H₅, OCH₃, OC₂H₅, OCF₃ stehen,
- R⁵: für H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1- Dimethylpropyl, 1,2-Dimethylpropyl, 2,2-Dimethylpropyl, 1-Ethylpropyl und Hexyl, jeweils unsubstituiert oder einfach oder mehrfach substituiert mit einem Substituenten ausgewählt aus der Gruppe bestehend aus F, Cl, Br, I, NH₂, SH und OH; oder für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF₃, Methoxy und Ethoxy;
- R⁶: für H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl und Hexyl; jeweils unsubstituiert oder einfach oder mehrfach substituiert mit einem Substituenten ausgewählt aus der Gruppe bestehend aus F, Cl, Br, I, NH₂, SH und OH; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF_{3,} Methoxy und Ethoxy; oder einen Rest gemäß Formel IIa steht: 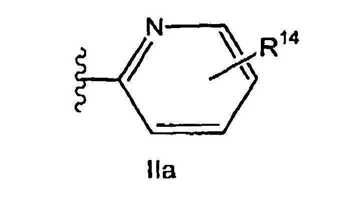
oder die Reste R⁵ und R⁶ zusammen einen Ring bilden und CH₂CH₂OCH₂CH₂, CH₂CH₂NR₁₁CH₂CH₂ oder (CH₂)₃₋₆ bedeuten,
- R⁷: für H, für einen Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, 1-Ethylpropyl und Hexyl; für einen Rest ausgewählt aus der Gruppe bestehend aus Cyclopropyl, 2-Methylcyclopropyl, Cyclopropylmethyl, Cyclobutyl, 2-Methylcyclobutyl, 3-Methylcyclobutyl, Cyclobutylmethyl, Cyclopentyl, Cyclohexyl, Cycloociyt; einen Acylrest C(O)R⁸ oder einen Sulfonylrest S(O₂)R⁹ steht,
- R⁸: für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
- R⁹: für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethytethyl, Pentyl, 1,1-Dimethytpropyl, 1,2-Dimethylpropyl, 2,2-Dimethylpropyl, Hexyl, 1-Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
- R¹¹: für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl oder für einen unsubstituierten Cycloalkyl-Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl; für einen Aryl-Rest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl oder einen unsubstituierten Heteroaryl-Rest ausgewählt aus der Gruppe bestehend aus Pyrrolyl, Furyl, Thienyl, Pyrazolyl, lmidazolyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, lsochinolinyl, Chinazolinyl, Carbazolyl, Phenazinyl und Phenothiazinyl, für einen über C₁₋₃-Alkylen gebundenen Aryl-Rest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl oder für einen über C₁₋₃-Alkylen gebundenen unsubstituierten Heteroaryl-Rest ausgewählt aus der Gruppe bestehend aus Pyrrolyl, Furyl, Thienyl, Pyrazolyl, Imidazolyl, Pyridinyl, Pyridazinyl, Pyrimidinyl, Pyrazinyl, Pyranyl, Indolyl, lndazolyl, Purinyl, Pyrimidinyl, lndolizinyl, Chinolinyl, lsochinolinyl, Chinazolinyl, Carbazolyl, Phenazinyl und Phenothiazinyl, Acyl C(O)R¹² oder Sulfonyl S(O₂)R¹³ steht,
- R¹²: für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl oder für einen Arylrest ausgewählt aus der Gruppe bestehend aus Phenyl, 3-Hydroxyphenyl, 3-Methoxyphenul, 2,3-Dihydroxyphenyl, 2,3-Dimethoxyphenyl und 1-Naphthyl steht,
- R¹³: für für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
- R14: für H, F, Br, I, Cl, OH, SH, NH₂, NO₂, CH₃, C₂H₅, OCH₃, OC₂H₅, OCF₃ steht;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
unter der Bedingung, dass die folgenden Verbindungen: 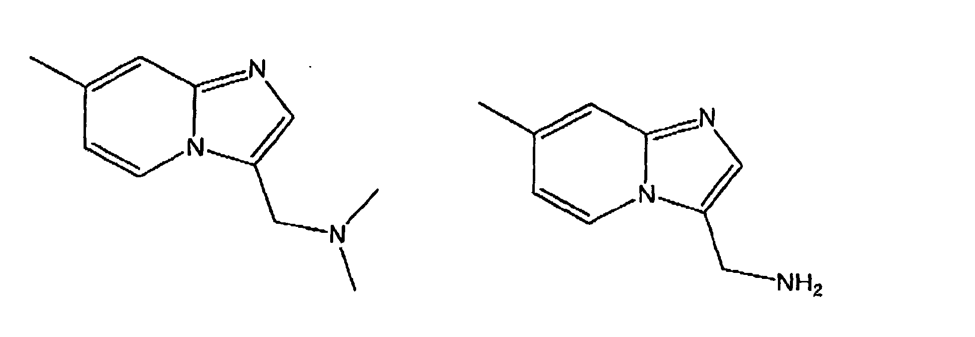 vom Schutz ausgenommen sind.

Der Ausdruck "C₁₋₁₂-Alkyl-Rest" umfaßt im Sinne der vorliegenden Erfindung acyclische gesättigte oder ungesättigte Kohlenwasserstoffreste, die verzweigt- oder geradkettig sowie unsubstituiert oder wenigstens einfach substituiert sein können, mit 1 bis 6 Kohlenstoff Atomen. Das heißt, es werden neben C₁₋₁₂-Alkanylen auch C₂-₁₂-Alkenyle und C₂₋₁₂-Alkinyle umfaßt, wobei die Alkenyle mindestens eine Kohlenstoff-Kohlenstoff Doppelbindung, die Alkinyle mindestens eine Kohlenstoff-Kohlenstoff Dreifachbindung aufweisen. Vorzugsweise ist der C₁₋₁₂-Alkyl-Rest ausgewählt aus der Gruppe Methyl, Ethyl, n-Propyl, 2-Propyl, n-Butyl, iso-Butyl, sec.-Butyl, tert.-Butyl, n-Pentyl, iso-Pentyl, neo-Pantyl, n-Hexyl, 2-Hexyl, n-Octyl, Ethenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl, Hexinyl, Octenyl und Octinyl.

Sofern der C₁₋₁₂Alkyl-Rest einfach oder mehrfach substituiert vorliegt, ist (sind) ein oder mehrere Wasserstoffrest(e) bevorzugt durch einen Substituenten ausgewählt aus der Gruppe F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH. S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 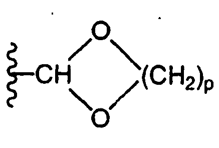 mit p = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂₋Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl und Heterocyclyl ersetzt, wobei unter mehrfach substituierten C₁₋₆-Alkyl-Resten solche Reste zu verstehen sind, die entweder an verschiedenen Atomen oder an demselben Atom des C₁₋₆₋Alkyl-Restes mehrfach, z.B. zwei- oder dreifach, substituiert sind, beispielsweise dreifach am gleichen Kohlenstoff-Atom wie im Falle von CF₃ oder -CH₂CF₃ oder an verschiedenen Atomen wie im Falle von -CH(OH)-CH=CH-CHCl₂. Die Mehrfachsubstitution kann mit dem gleichen oder mit verschiedenen Substituenten erfolgen. Sofern der Substituent selbst eine Alkylgruppe aufweist, ist diese bevorzugt ausgewählt aus der Gruppe Methyl, Ethyl, CH₂-OH und CF₃.

Der Ausdruck "C₃₋₈-Cycloalkyl-Rest" umfaßt für die Zwecke der vorliegenden Erfindung cyclische Kohlenwasserstoffe mit 3 bis 8 Kohlenstoffatomen, die gesättigt oder ungesättigt, unsubstituiert oder wenigstens einfach substituiert sein können, wobei die Bindung des Cycloalkyl-Restes an das Grundgerüst der allgemeinen Formel I über jedes beliebige Ringglied des Cycloalkyl-Restes erfolgen kann. Bevorzugt ist der C₃₋₈-Cycloalkyl-Rest ausgewählt aus der Gruppe Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl. Besonders bevorzugt ist der C₃₋₈-Cycloalkyl-Rest ein Cyctohexyl-Rest.

Der Ausdruck "Aryl-Rest" bedeutet im Sinne der vorliegenden Erfindung aromatische Kohlenwasserstoffe, die auch mit weiteren gesättigten, zumindest teilweise ungesättigten oder aromatischen Ringsystemen kondensiert sein können, wobei die Bindung des Aryl-Restes an das Grundgerüst der allgemeinen Formel I über jedes beliebige Ringglied des Aryl-Restes erfolgen kann. Sofern der Aryl-Rest mehr als einen Substituenten aufweist, können diese gleich oder verschieden sein und in jeder beliebigen und möglichen Position des Aryl-Restes vorliegen. Vorzugs.weise ist der Aryl-Rest ausgewählt aus der Gruppe von unsubstituierten oder wenigstens einfach substituiertem Phenyl, Anthracenyl, 1-Naphthyl und 2-Naphthyl. Besonders bevorzugt ist der Aryl-Rest ausgewählt aus der Gruppe Phenyl, 3-Hydroxyphenyl, 3-Methoxyphenyl, 2,3-Dihydroxyphenyl, 2,3-Dimethoxyphenyl und 1-Naphthyl.

Sofern der C₃₋₈-Cycloalkyl oder der Aryl-Rest einfach oder mehrfach substituiert ist, wir darunter bevorzugt die ein- oder mehrfache, z.B. zwei-, drei- oder vierfache, Substitution eines oder mehrerer Wasserstoffatome des Ringsystems mit einem Substituenten ausgewählt aus der Gruppe F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆₋Alkyl-Aryl, 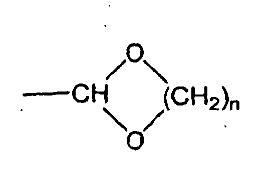 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂Alkyl, CO₂₋Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂₋Aryl, SO₂NH₂, SO₃H, CF₃, =O, =S; Alkyl, Cycloalkyl, Aryl, Heteroaryl und Heterocyclyl verstanden, wobei ein Substituent ggf. seinerseits substituiert sein kann. Die Mehrfachsubstitution erfolgt dabei mit gleichen oder unterschiedlichen Substituenten. Für "Aryl-Reste" sind besonders bevorzugte Substituenten ausgewählt aus der Gruppe F, CF₃, OH und O-CH₃. Für "Cycloalkyl-Reste" sind besonders bevorzugte Substituenten CO₂H oder CO₂Ethyl.

Der Ausdruck "Heteroaryl" steht im Sinne der vorliegenden Erfindung für einen 5-, 6- oder 7-gliedrigen cyclischen aromatischen Rest, der mindestens 1, ggf. auch 2, 3, 4 oder 5 Heteroatome, aufweist, wobei die Heteroatome gleich oder verschieden sein können und wobei die Bindung an das Grundgerüst der allgemeinen Formel I über jedes beliebige und mögliche Ringglied des Heteroaryl-Restes erfolgen kann. Sofern der Heteroaryl-Rest mehr als einen Substituenten aufweist, können diese Heteroarylsubstituenten gleich oder verschieden sein und in jeder beliebigen und möglichen Position des Heteroaryls vorhanden sein. Der Heterocyclus kann auch mit weiteren gesättigten, zumindest teilweise ungesättigten oder aromatischen Ringsystemen kondensiert sein. Bevorzugte Heteroatome sind ausgewählt aus der Gruppe Stickstoff, Sauerstoff und Schwefel. Vorzugsweise ist der Heteroaryl-Rest ausgewählt aus der Gruppe von unsubstituiertem oder wenigstens einfach substituiertem Pyrrolyl, Furyl, Thienyl, Pyrazolyl, Imidazolyl, Pyridinyl, Pyridazinyl, Pyrimidinyl, Pyrazinyl, Pyranyl, lndolyl, Indazolyl, Purinyl, Pyrimidinyl, Indolizinyl, Chinolinyl, lsochinolinyl, Chinazolinyl, Carbazolyl, Phenazinyl und Phenothiazinyl. Besonders bevorzugte Heteroaryl-Reste sind ausgewählt aus der Gruppe Pyridin-2-yl, Pyridin-3-yl, Furan-2-yl, Furan-3-yl, 5-Hydroxymethylen-furan-2-yl, 5-Nitro-furan-2-yl, 5-[1,3]-dioxolan-furan-2-yl, 5-Carbonsäure-furan-2-yl; Thien-2-yl (2-Thiophen), Thien-3-yl (3-Thiophen) und 5-Carbonsäure-2-thiophen (5-Carbonsäurethien-2-yl).

Der Ausdruck "Heterocyclyl" umfaßt im Sinne der vorliegenden Erfindung einen 3-, 4-, 5-, 6- oder 7-gliedrigen cyclischen organischen Rest, der mindestens 1, ggf. auch 2, 3, 4 oder 5 Heteroatome im Ringsystem aufweist; wobei die Heteroatome gleich oder verschieden sein können und der cyclische Rest gesättigt oder ungesättigt, nicht aber aromatisch ist und unsubstituiert oder wenigstens einfach substituiert sein kann. Die Bindung des Heterocyclyl-Restes an das Grundgerüst der allgemeinen Formel I kann über jedes beliebige Ringglied des Heterocyclyl-Restes erfolgen. Der Heterocyclyl-Rest kann auch Teil eines bi- oder polycyclischen Systems sein. Bevorzugte Heteroatome sind ausgewählt aus der Gruppe Stickstoff, Sauerstoff und Schwefel. Vorzugsweise ist der C₃₋₇-Heterocyclyl-Rest ausgewählt aus der Gruppe Tetrahydrofuryl, Tetrahydropyranyl, Pyrrolidinyl, Piperidinyl, Piperazinyl und Morpholinyl.

Sofern die erfindungsgemäßen Verbindungen der allgemeinen Formel I oder deren physiologisch verträgliche Salze wenigstens ein Asymmetriezentrum aufweisen, können sie in Form ihrer Racemate, ihrer reinen Enantiomeren, ihrer reinen Diastereomeren oder in Form eines Gemisches aus wenigstens zwei der vorstehend genannten Stereoisomeren vorliegen. Ebenso können die substituierten C-Imidazo[1,2-a]pyridin-3-yl-methylamine der allgemeinen Formel I auch in Form von Mischungen ihrer Enantiomeren oder Diastereomeren vorliegen. Diese Mischungen können die jeweiligen Stereoisomeren in jedem beliebigen Mischungsverhältnis aufweisen. Bevorzugt werden chirale substituierte C-Imidazo[1,2-a]pyridin-3-yl-methylamine der allgemeinen Formel I in enantiomerenreiner Form verwendet.

Im Sinne dieser Erfindung versteht man auch unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C₂-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4- oder C5-Alkyl, C₁₋₅-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄₋Cycloalkyl für C3- oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇₋Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Diöxolan.

Dabei versteht man im Zusammenhang mit Alkyl und Cycloalkyl - solange dies nicht ausdrücklich anders definiert ist - unter dem Begriff substituiert im Sinne dieser Erfindung auch die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂, -CH₂-CH₂-CH₂CH₂-, -CH₂₋CH₂-CH₂-CH₂-CH₂- und CH₂-CH₂-CH₂-CH₂-CH₂-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, unter (CH₂)₄₋₅ ist -CH₂-CH₂-CH₂-CH₂- und -CH₂-CH₂-CH₂-CH₂₋CH₂- zu verstehen, etc.

Unter einem Aryl-Rest werden auch Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden auch heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert auch die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über eine C₁₋₃₋Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über eine C₁₋₃-AlkylenGruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist im Sinne dieser Erfindung jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes (insbesondere mit Kationen oder Basen) versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes (insbesondere mit Anionen oder Säuren) versteht man im Sinne dieser Erfindung weiter Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Geeignete Salze im Sinne dieser Erfindung und in jeder beschriebenen Verwendung und jedem der beschriebenen Arzneimittel sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Bevorzugte Ausführungsformen der erfindungsgemäßen Verbindungen der C-Imidazo[1,2-a]pyridin-3-yl-methylamine sind den Unteransprüchen zu entnehmen.

Die Herstellung der substituierten C-Imidazo[1,2-a]pyridin-3-yl-methylamine der allgemeinen Formel 1 kann nach üblichen, dem Fachmann bekannten Methoden erfolgen.

Vorzugsweise erfolgt die Herstellung der erfindungsgemäßen Verbindungen der allgemeinen Formel 1 schrittweise, im ersten Schritt durch Umsetzung eines substituierten 2-Aminopyridins der allgemeinen Formel III, worin R¹ und R² die Bedeutung gemäß der oben angegebenen allgemeinen Formel I haben, 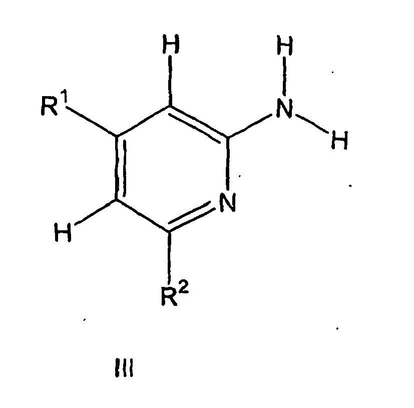 vorzugsweise in Lösung mit einer α-Halogencarbonylverbindung der allgemeinen Formel IV, 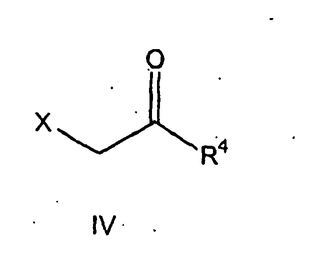 worin die Reste R³ und R⁴ die Bedeutung gemäß der allgemeinen Formel haben und X für Halogen, vorzugsweise für Cl, Br oder I steht, unter Abspaltung von Wasser und Halogenwasserstoff und Bildung des lntermediats der Formel V in 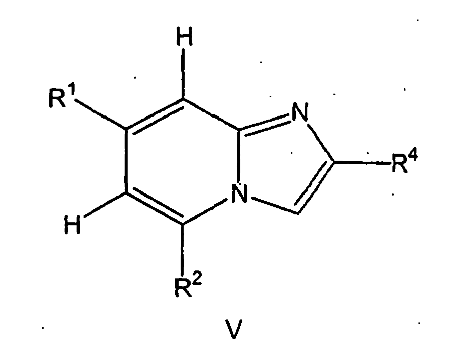 und anschließende Aminomethylierung dieses Intermediats V in einem zweiten Schritt.

Vorteilhafterweise wird der erste Verfahrensschritt unter Bedingungen durchgeführt, bei denen Wasser und/oder Halogenwasserstoff vorzugsweise kontinuierlich aus dem Reaktionsgemisch entfernt werden.

Halogenwasserstoff kann bevorzugt durch Zugabe löslicher oder unlöslicher organischer oder anorganischer Basen gebunden und so aus dem Reaktionsgemisch entfernt werden.
Wasser kann bevorzugt durch azeotrope Destillation oder durch Zusatz von Trockenmitteln oder hygroskopischen Substanzen aus dem Reaktionsgemisch entzögen werden.

Die Herstellung der erfindungsgemäßen Intermediate der allgemeinen Formel V nach dem obenstehenden Verfahren, mit oder ohne Lösungsmittel, bei Temperaturen von mehr als 100 °C stellt eine weitere Möglichkeit dar, Wasser aus dem Reaktionsgemisch zu entfernen.

Besonders bevorzugt ist die Herstellung der erfindungsgemäßen Intermediate der allgemeinen Formel V durch Umsetzung substituierter 2-Aminopyridine der allgemeinen Formel III mit α-Halogencarbonylverbindungen der allgemeinen Formel IV, worin X für Br steht, in siedendem, wasserfreien Ethanol.

Ebenfalls bevorzugt ist die Herstellung der erfindungsgemäßen Intermediate der allgemeinen Formel V durch Umsetzung substituierter 2-Aminopyridine der allgemeinen Formel III mit (α-Halogencarbonylverbindungen der allgemeinen Formel IV, worin X für Br oder Cl steht, in siedendem, wasserfreien Di- und/oder Trichlormethan am Wasserabscheider.

Die substituierten 2-Aminopyridine der allgemeinen Formel III sowie die α-Halogencarbonylverbindungen der allgemeinen Formel IV sind allgemein am Markt erhältlich oder können nach üblichen, dem Fachmann bekannten Methoden hergestellt werden.

Der zweite Verfahrensschritt ist die Aminomethylierung der erfindungsgemäßen Intermediate der allgemeinen Formel V durch Umsetzung mit Iminiumsalzen der Formel VI, die sowohl zuvor separat als auch in situ hergestellt werden können.

Die Imminiumsalz der allgemeinen Formel VI, 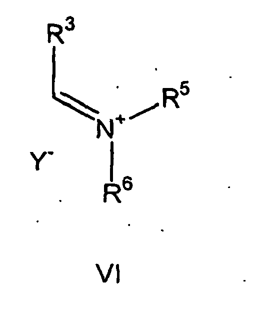 worin Y vorzugsweise Cl⁻, AlCl₄⁻, Br⁻ oder I⁻ bedeutet, können nach literaturbekannten Verfahren durch Umsetzung von Aminalen der allgemeinen Formel VII 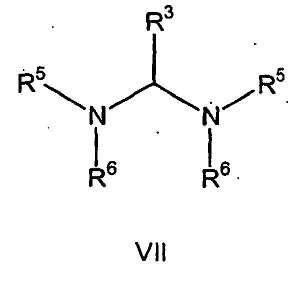 mit Säurechloriden, beispielsweise Acetylchlorid oder Thionylchlorid, hergestellt (Houben-Weyl - Methoden der Organischen Chemie, E21b, 1995, S. 1925-1929).

Die Imminiumsalze der allgemeinen Formel VI müssen dabei nicht isoliert werden, sondern können in situ mit erfindungsgemäßen Intermediaten der allgemeinen Formel V zu erfindungsgemäßen Substanzen der allgemeinen Formel I umgesetzt werden.

Zur Einführung eines Dimethylaminomethylrest ist außerdem die Umsetzung erfindungsgemäßer Intermediate der allgemeinen Formel V mit Paraformaldehyd und Dimethylamrnoniumchlorid bei Temperaturen zwischen 50 und 150 °C ein geeignetes Verfahren.

Die erfindungsgemäßenen substituierten C-Imidazo[1,2-a]pyridin-3-yl-methylamine der allgemeinen Formel I können nach dem zu ihrer Herstellung eingesetzten Verfahren sowohl als freie Base wie auch als Salz isoliert werden. Die freie Base der jeweiligen Verbindung der allgemeinen Formel I wird üblicherweise nach erfolgter Umsetzung gemäß dem oben beschriebenen erfindungsgemäßen Verfahren und ggf. anschließender Aufarbeitung nach üblichen, dem Fachmann bekannten Methoden erhalten. Die so erhaltene oder in-situ ohne Isolierung gebildete freie Base der jeweiligen Verbindung der allgemeinen Formel I kann dann, beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure; Mandelsäure, Fumarsäure, Milchsäure, Citronensäure, Glutaminsäure oder Asparaginsäure, in das entsprechende, physiologisch verträgliche Salz übergeführt werden.

Die Überführung der jeweiligen Verbindung der allgemeinen Formel 1 kann bevorzugt auch durch Versetzen der in einem geeigneten organischen Lösungsmittel, wie z.B. Butan-2-on (Methylethylketon), gelösten Verbindung der allgemeinen Formel 1 als freie Base mit Trimethylsilylchlorid (TMSCI) erhalten werden.

Sofern die erfindungsgemäßen substituierte C-Imidazo[1,2-a]pyridin-3-yl-methylamine der allgemeinen Formel 1 nach dem erfindungsgemäßen Herstellungsverfahren in Form ihrer Racemate oder anderer Mischungen ihrer verschiedenen Enantiomeren und/oder Diastereomeren erhalten wird, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Ein weiterer Gegenstand der vorliegenden Erfindung ist die Verwendung wenigstens eines substituierten C-Imidazo[1,2-a]pyridin-3-yl-methylamins der oben angebenen allgemeinen Formel I als Inhibitor der Stickstoffmonoxid-Synthase zur Herstellung eines Arzneimittels zur Behandlung von Migräne, septischem Schock, neurodegenerativen Erkrankungen, wie Multipler Sklerose, Morbus Parkinson, Morbus Alzheimer oder Morbus Huntington, Entzündungsschmerz, cerebraler Ischämie, Diabetes, Meningitis, Arteriosklerose, Krebserkrankungen oder zur Wundheilung.

Die entsprechenden Arzneimittel können als flüssige, halbfeste oder feste Arzneiformen, beispielsweise in Form von Injektionslösungen, Tropfen, Säften, Sirupen, Sprays, Suspensionen, Granulaten, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einem erfindungsgemäßen substituierten C-Imidazo[1,2-a]pyridin-3-yl-methylamin der allgemeinen Formel 1 enthalten die erfindungsgemäßen Arzneimittel üblicherweise weitere physiologisch verträgliche pharmazeutische Hilfsstoffe, die bevorzugt ausgewählt sind aus der Gruppe von Trägermaterialien, Füllstoffen, Lösungsmittel, Verdünnungsmittel, oberflächenaktiven Stoffen, Farbstoffen, Konservierungsstoffen, Sprengmittel, Gleitmittel, Schmiermittel, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäßen Verbindungen der allgemeinen Formel 1 in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel 1 auch verzögert freisetzen.

Die Herstellung der Arzneimittel erfolgt mit Hilfe von üblichen, dem Fachmann bekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt somit als Teil der Offenbarung.

Die an den Patienten zu verabreichende Menge der jeweiligen Verbindung der allgemeinen Formel 1 kann variieren und ist beispielsweise abhängig vom Gewicht oder dem Alter des Patienten sowie von der Applikationsart, der Indikation und dem Schweregrad der Erkrankung. Üblicherweise werden 0,1 bis 5000 mg/kg, vorzugsweise 1 bis 500 mg/kg, besonders bevorzugt 2 bis 250 mg pro kg Körpergewicht des Patienten wenigstens einer Verbindung der allgemeinen Formel I appliziert.

Im folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1:

### Synthese von 2,7-Dimethyl-imidazo[1,2-α]pyridin-3-ylmethyl)dimethyl-amin

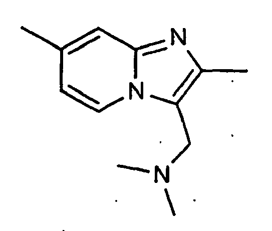

### (2,7-Dimethyl-imidazo[1,2-a]pyridin-3-ylmethyl)-dimethyl-amin

Zu 0,83 g Formaldehyd (Formalin 37 %-tig) und 1,30 ml Dimethylamin (40 %-tig in Wasser) in 1,41 ml Eisessig gab man bei 0 °C unter Stickstoffatmosphäre 1,50 g 2,7-Dimethyl-imidazo[1,2-α]pyridin, erhitzte das Reaktionsgemisch zwei Stunden bei 50 °C und rührte über Nacht bei einer Temperatur von 20 bis 25 °C. Zur weiteren Reinigung wurde das Reaktionsgemisch mit 10 %-iger Natronlauge alkalisch gestellt und mit Diethylether extrahiert. Die organischen Phasen wurden vereinigt und über Natriumsulfat getrocknet. Nach Entfernung des organischen Lösungsmittels durch Destillation wurden 1,70 g des Rohproduktes erhalten. Nach Reinigung durch Säulenchromatographie erhielt man 177 mg des Produktes als farbloses Öl. 177 mg der Base wurden mit 1 ml Ethylmethylketon verdünnt und durch Zugabe von 0,009 ml Wasser und 0,121 ml Chlortrimethylsilan und anschließendem Rühren über Nacht als Hydrochlorid gefällt. Man erhielt 170 mg des 2,7-Dimethyl-imidazo[1,2-α]pyridin-3-ylmethyl)-dimethyl-amin-hydrochlorids (entsprechen 6,5 % der theoretischen, Menge) als farblosen Feststoff.

### Beispiel 2:

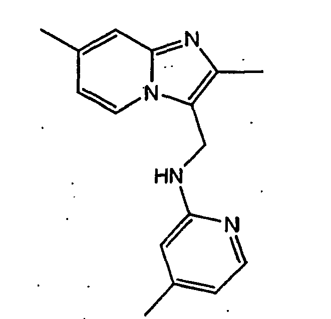

### (2,7-Dimethyl-imidazo[1,2-a]pyridin-3-ylmethyl)-(4-methy(-pyridin-2-yl)-amin

### 1.Stufe: Synthese von 2,7-Dimethyl-imidazo[1,2-a]pyridin-3-carbonsäureethylester

Zu einer Lösung von 10 g 2-Amino-4-methylpyridin in 200 ml Ethanol gab man 15,2 g Ethyl-2-Chloracetat, rührte für 8 Stunden am Rückfluß und ließ die Reaktionslösung über Nacht bei Raumtemperatur rühren. Nach Entfernen des Lösungsmittels wurde der Rückstand mit wässeriger 10% iger HCl aufgenommen, mit Dichlormethan gewaschen, mit Natriumhydrogencarbonat basisch gestellt und mit Dichlormethan extrahiert. Die vereinigten organischen Phasen wurden mit Wasser gewaschen und über Natriumsulfat getrocknet. Nach Entfernung des Lösungsmittels und säulenchromatographischer Aufreinigung erhielt man das Produkt in einer Ausbeute von 35%.

### 2. Stufe: Synthese von 2,7-Dimethyl-imidazo[1,2-a]pyridin-3-carbonsäure (4-methyl-pyridin-2-yl)-amid

Zu einer Lösung von 3,2 g 2-Amino-4-methylpyridin in 57 ml THF wurden unter Rühren bei 0°C 37 ml n-BuLi (1.6M in Hexan) hinzugetropft. Nach Erwärmen auf Raumtemperatur rührte man für eine Stunde. Die Reaktionslösung wurde auf -78°C abgekühlt, 5,8 g 2,7-Dimethyl-imidazo[1,2-a]pyridine-3-carbonsäureethylester gelöst in 10 ml THF langsam zugetropft und für eine Stunde gerührt. Nach Zugabe von 60 ml Ammoniumchlorid bei Raumtemperatur wurde mit Dichlormethan extrahiert, die organischen Phasen über Natriumsulfat getrocknet und das Lösungsmittel entfernt. Das Produkt wurde durch Aufkochen und Fällung in Hexan aufgereinigt und mit einer Ausbeute von 68% erhalten.

### 3. Stufe: Synthese von (2,7-Dimethyl-imidazo[1,2-a]pyridin-3-ylmethyl)-(4-methyl-pyridin-2-yl)-amin

Zu einer Suspension von 160 mg Lithiumaluminiumhydrid in 12 ml Tetrahydrofuran gab man bei Raumtemperatur 760 mg 2,7-Dimethyl-imidazo[1,2-a]pyridine-3-carbonsäure (4-methyl-pyridin-2-yl)-amid. Anschließend wurde 2.5 Stunden am Rückfluß erhitzt. Nach Zugabe einer wässerigen KOH-Lösung wurde die entstehende Suspension nochmals für 15 Minuten am Rückfluß erhitzt. Nach Filtration, Destillation des Lösungsmittels und säulenchromatographischer Aufreinigung erhielt man das Produkt mit einer Ausbeute von 25%.

### Molekularpharmakologische Untersuchung:

Der IC50-Wert der Beispielverbindungen wurde in einem Citrullin-Assay bestimmt. Dieser Assay wurde wie von D. S. Bredt und S. H. Snyder (Proc. Natl. Acad. Sci. USA (1990), 87, 682-685) beschrieben durchgeführt. Die Ergebnisse von Beispielverbindungen im Citrullin-Assay sind in Tabelle 2 wiedergegeben.

**Tabelle 2:**

| Beispiel-Nr.: | Hemmung der Stickstoffmonoxid-Synthase IC50 [µm] |
|---|---|
| 1 | 4,0 |
| 2 | 2,5 |

## Patentansprüche

1. Substituiertes C-Imidazo[1,2-a]pyridin-3-yl-methylamin der allgemeinen Formel I, 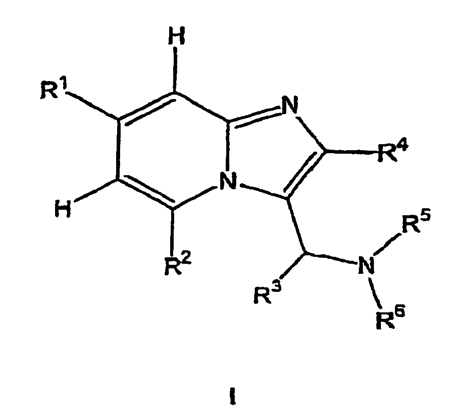 worin jeweils
R¹ für OH, SH, NH₂, NHCH₃, N(CH₃)₂, CH₃, CH₂Cl, CH₂F, CHF₂, CF₃, OCH₃, OCH₂Cl, OCH₂F, OCHF₂, OCF₃, C₂H₅, CHClCH₃, CH₂CH₂Cl, CHFCH₃, CH₂CH₂F, CH₂CHF₂, CH₂CF₃, OC₂H₅, COOH, CH₂OH, CHOHCH₃ oder CH₂CH₂OH steht,
R² für H oder CH₃ steht,
R³ für H steht
R⁴ für H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1- Ethylpropyl, Hexyl, Heptyl, Octyl, Nonyl, Decyl, Undecyl und Dodecyl; jeweils unsubstituiert oder ein- oder mehrfach substituiert mit einem Substituenten ausgewählt aus der Gruppe bestehend aus F, Cl, Br, I, NH₂, SH und OH; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF₃, Methoxy und Ethoxy; oder einen Rest gemäß Formel II steht, 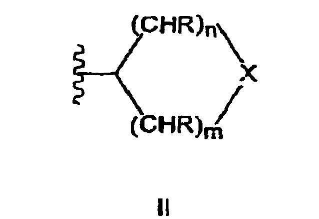
worin jeweils
n für eine Zahl zwischen 0 und 6 steht,
m für eine Zahl zwischen 0 und 6 steht,
1 ≤ m+n ≤ 6 ist,
X für O, S, SO, SO₂ oder NR⁷ steht,
R unabhängig voneinander für H, F, Br, I, Cl, OH, SH, NH₂, NO₂, CH₃, C₂H₅, OCH₃, OC₂H₅, OCF₃ stehen,
R⁵ für H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl und Hexyl, jeweils unsubstituiert oder einfach oder mehrfach substituiert mit einem Substituenten ausgewählt aus der Gruppe bestehend aus F, Cl, Br, I, NH₂, SH und OH; oder für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF₃, Methoxy und Ethoxy;
R⁶ für H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl und Hexyl; jeweils unsubstituiert oder einfach oder mehrfach substituiert mit einem Substituenten ausgewählt aus der Gruppe bestehend aus F, Cl, Br, I, NH₂, SH und OH; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF₃, Methoxy und Ethoxy; oder einen Rest gemäß Formel IIa steht: 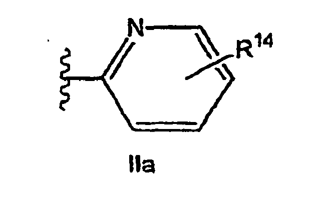
oder die Reste R⁵ und R⁶ zusammen einen Ring bilden und CH₂CH₂OCH₂CH₂, CH₂CH₂NR¹¹CH₂CH₂ oder (CH₂)3-6 bedeuten,
R⁷ für H, für einen Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, 1-Ethylpropyl und Hexyl; für einen Rest ausgewählt aus der Gruppe bestehend aus Cyclopropyl, 2-Methylcyclopropyl, Cyclopropylmethyl, Cyclobutyl, 2-Methylcyclobutyl, 3-Methylcyclobutyl, Cyclobutylmettlyl, Cyclopentyl, Cyclohexyl, Cyclooctyl; einen Acylrest C(O)R⁸ oder einen Sulfonylrest S(O₂)R⁹ steht,
R⁶ für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1 -Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
R⁹ für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethytpropyl, 1,2-Dimethylpropyl, 2,2-Dimethylpropyl, Hexyl, 1-Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
R¹¹ für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl oder für einen unsubstituierten Cycloalkyl-Rest ausgewählt aus der Gruppe bestehend aus Cyclapropyl, Cyclobutyl, Cyclopentyl, Cyclohexyl, Cycloheptyl, Cyclooctyl, Cyclopentenyl, Cyclohexenyl, Cycloheptenyl und Cyclooctenyl; für einen Aryl-Rest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl oder einen unsubstituierten Heteroaryl-Rest ausgewählt aus der Gruppe bestehend aus Pyrrolyl. Furyl, Thienyl, Pyrazolyl, Imidazolyl, Pyridinyl, Pyridazinyl, Pyrimidinyl, Pyrazinyl, Pyranyl, Indolyl, lndazolyl, Purinyl, Pyrimidinyl, Indolizinyl, Chinollnyl, Isochinolinyl, Chinazolinyl, Carbazolyl, Phenazinyl und Phenothiazinyl, für einen über C₁₋₃-Alkylen gebundenen Aryl-Rest ausgewählt aus der Gruppe bestehend aus Phenyl, 3-Hydroxyphenyl, 3-Methoxyphenyl, 2,3-Dihydroxyphenyl, 2.3-Dimethoxyphenyl und 1-Naphthyl oder für einen über C₁₋₃-Alkylen gebundenen unsubstituierten Heteroaryl-Rest ausgewählt aus der Gruppe bestehend aus Pyrrolyl, Furyl, Thienyl, Pyrazolyl, Imidazolyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, lsochinolinyl, Chinazolinyl, Carbazolyl, Phenazinyl und Phenothiazinyl, Acyl C(O)R¹² oder Sulfonyl S(O₂)R¹³ steht,
R¹² für H, für einen unsubstituierten Alkyl-Rest ausgewählt aus der Gruppe bestehend aus Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1 -Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
R¹³ für H, für einen unsubstituierten Alkyl-Rest ausgewählt aus der Gruppe bestehend aus Methyl, Ethyl, Propyl, Methylethyl, Butyl, 1-Mathylpropyl, 2-Methylpropyl, 1,1-Dimethylethyl, Pentyl, 1,1-Dimethylpropyl, 1,2-Dimethylpropyl, 2,2-Dimethytpropyl, Hexyl, 1-Methylpentyl oder für einen Arylrest ausgewählt aus der Gruppe bestehend aus Phenyl, 3-Hydroxyphenyl, 3-Methoxyphenyl, 2,3-Dihydroxyphenyl, 2,3-Dimethoxyphenyl und 1-Naphthyl steht,
R¹⁴ für H, F, Br, I, Cl, OH, SH, NH₂, NO₂, CH₃, C₂H₅, OCH₃, OC₂H₅, OCF₃ steht;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
unter der Bedingung, dass die folgenden Verbindungen: 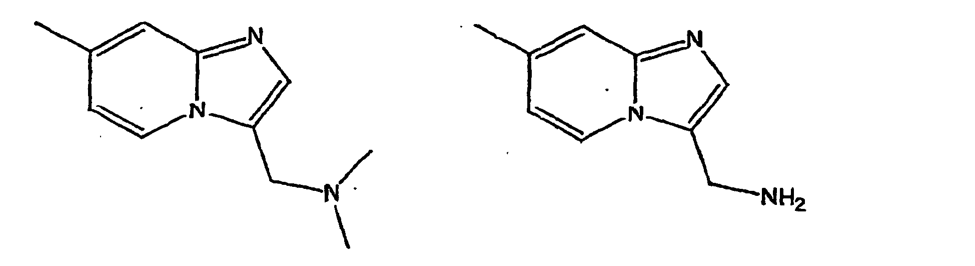 vom Schutz ausgenommen sind.

2. C-Imidazo[1,2-a]pyridin-3-yl-methylamin gemäß Anspruch 1, **dadurch gekennzeichnet, daß** R⁴
für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2.2-Dimethylpropyl, 1-Ethylpropyl, Hexyl, Heptyl, Octyl, Nonyl, Decyl, Undecyl und Dodecyl; jeweils unsubstituiert oder ein- oder mehrfach substituiert mit einem Substituenten ausgewählt aus der Gruppe bestehend aus F, Cl, Br, I, NH₂, SH und OH; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, jeweils unsubstituiert oder einfach oder mehrfach substituiert mit einem Substituenten ausgewählt aus der Gruppe bestehend aus Methyl, Ethyl, n-Propyl, i-Propyl, CF₃, Methoxy und Ethoxy, oder einen Rest gemäß Formel II steht, 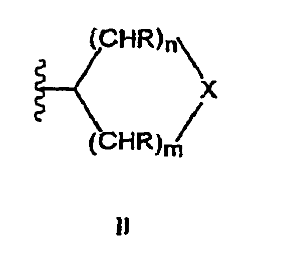 worin jeweils
n für eine Zahl zwischen 0 und 6 steht,
m für eine Zahl zwischen 0 und 6 steht,
1 ≤ m+n ≤ 6 ist,
X O, S, SO, SO₂ oder NR⁷ steht,
R unabhängig voneinander für H, F, Br, I, Cl, OH, SH, NH₂, NO₂. CH₃, C₂H₅, OCH₃, OC₂H₅, OCF₃ stehen.

3. C-Imidazo[1,2-a]pyridin-3-yl-methylamin Anspruch 1 oder 2, **dadurch gekennzeichnet, daß** R¹ für C₂H₅,CH₃ oder CF₃, vorzugsweise CH₃, steht.

4. C-imidazo[1,2-a]pyridin-3-yl-methylamin gemäß einem der Ansprüche 1 bis 3, **dadurch gekennzeichnet, daß** R⁵ für
H; für einen Rest ausgewählt aus der Gruppe bestehend aus Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert-Butyl, 1-Methylbutyl, 2-Methylbutyl. 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl und Hexyl, jeweils unsubstituiert oder einfach oder mehrfach substituiert mit einem Substituenten ausgewählt aus der Gruppe bestehend aus F, Cl, Br, I, NH₂, SH und OH, steht

5. C-Imidazo[1,2-a]pyridin-3-yl-methylamin gemäß einem der Ansprüche 1 bis 4, **dadurch gekennzeichnet, daß** R² für H steht.

6. C-lmidazo[1,2-a]pyridin-3-yl-methylamin gemäß Anspruch 1:
2,7-Dimethyl-imidazo[1,2-a]pyridin-3-ylmethyl)-dimethyl-amin
(2,7-Dimethyl-imidazo[1,2-a]pyridin-3-ylmethyl)-4-methyl-pyridin-2-yl)-amin.

7. Arzneimittel enthaltend mindestens ein C-Imidazo[1,2-a]pyridin-3-yl-methylamin gemäß einem der Ansprüche 1 bis 6 sowie gegebenenfalls weitere Hilfsstoffe.

8. Verwendung einer Verbindung gemäß einem der Ansprüche 1 bis 6 zur Herstellung eines Arzneimittels zur Behandlung von Migräne, septischem Schock, neurodegenerativen Erkrankungen, vorzugsweise Multipler Sklerose, Morbus Parkinson, Morbus Alzheimer oder Morbus Huntington, Entzündungsschmerz, cerebraler Ischämie, Diabetes, Meningitis, Arteriosklerose, Krebserkrankungen oder zur Wundheilung.

## Claims

1. Substituted C-imidazo[1,2-a]pyridin-3-yl-methylamine of the general formula I 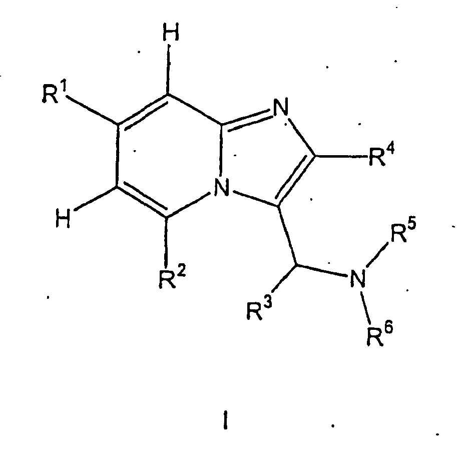 wherein in each case
R¹ represents OH, SH, NH₂, NHCH₃, N(CH₃)₂, CH₃, CH₂Cl, CH₂F , CHF₂, CF₃, OCH₃, OCH₂Cl, OCH₂F, OCHF₂, OCF₃, C₂H₅, CHClCH₃, CH₂CH₂Cl, CHFCH₃, CH₂CH₂F, CH₂CHF₂, CH₂CF₃, OC₂H₅, COOH, CH₂OH, CHOHCH₃ or CH₂CH₂OH,
R² represents H or CH₃,
R³ represents H;
R⁴ represents H; a radical chosen from the group consisting of methyl, ethyl, methylethyl, 1,1-dimethylethyl, propyl, 2-methylpropyl, 1-methylpropyl, 1-ethylpropyl, butyl, i-butyl, n-butyl, tert-butyl, 1-methylbutyl, 2-methylbutyl, 3-methylbutyl, 2,2-dimethylbutyl, pentyl, 2- methylpentyl, 3-methylpentyl, 1,1-dimethylpropyl, 1,2-dimethylpropyl, 2,2-dimethylpropyl, 1-ethylpropyl, hexyl, heptyl, octyl, nonyl, decyl, undecyl and dodecyl; in each case unsubstituted or mono- or polysubstituted by a substituent chosen from the group consisting of F, Cl, Br, I, NH₂, SH and OH; a radical chosen from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl, in each case unsubstituted or mono- or polysubstituted by a substituent chosen from the group consisting of methyl, ethyl, n-propyl, i-propyl, CF₃, methoxy and ethoxy; or a radical according to formula II 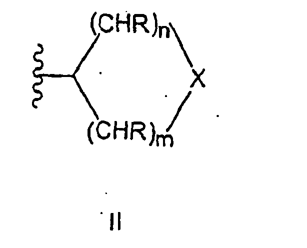
wherein in each case
n represents a number between 0 and 6,
m represents a number between 0 and 6,
1 ≤ m+n ≤ 6,
X represents O, S, SO, SO₂ or NR⁷,
R independently of one another represent H, F, Br, I, Cl, OH, SH, NH₂, NO₂, CH₃, C₂H₅, OCH₃, OC₂H₅, OCF₃,
R⁵ represents H; a radical chosen from the group consisting of methyl, ethyl, methylethyl, 1,1-dimethylethyl, propyl, 2-methylpropyl, 1-methylpropyl, 1-ethylpropyl, butyl, i-butyl, n-butyl, tert-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl and hexyl, in each case unsubstituted or mono- or polysubstituted by a substituent chosen from the group consisting of F, Cl, Br, I, NH₂, SH and OH; or a radical chosen from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl, in each case unsubstituted or mono- or polysubstituted by a substituent chosen from the group consisting of methyl, ethyl, n-propyl, i-propyl, CF₃, methoxy and ethoxy;
R⁶ represents H; a radical chosen from the group consisting of methyl, ethyl, methylethyl, 1,1-dimethylethyl, propyl, 2-methylpropyl, 1-methylpropyl, 1-ethylpropyl, butyl, i-butyl, n-butyl, tert-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1- ethylpropyl and hexyl; in each case unsubstituted or mono- or polysubstituted by a substituent chosen from the group consisting of F, Cl, Br, I, NH₂, SH and OH; a radical chosen from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl, in each case unsubstituted or mono- or polysubstituted by a substituent chosen from the group consisting of methyl, ethyl, n-propyl, i-propyl, CF₃, methoxy and ethoxy; or a radical according to formula IIa 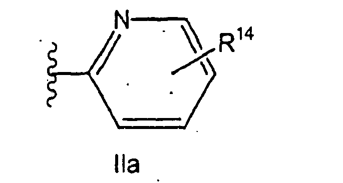
or the radicals R⁵ and R⁶ together form a ring and denote CH₂CH₂OCH₂CH₂, CH₂CH₂NR¹¹CH₂CH₂ or (CH₂)₃₋₆,
R⁷ represents H, a radical chosen from the group consisting of methyl, ethyl, propyl, methylethyl, butyl; 1-methylpropyl, 2-methylpropyl, 1,1-dimethylethyl, pentyl, 1,1-dimethylpropyl, 1,2-dimethylpropyl, 2,2-dimethylpropyl, 1-ethylpropyl and hexyl; a radical chosen from the group consisting of cyclopropyl, 2-methylcyclopropyl, cyclopropylmethyl, cyclobutyl, 2-methylcyclobutyl, 3-methylcyclobutyl, cyclobutylmethyl, cyclopentyl, cyclohexyl, cyclooctyl; an acyl radical C(O)R⁸ or a sulfonyl radical S(O₂)R⁹,
R⁸ represents H, an unsubstituted alkyl radical chosen from the group consisting of methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, or an aryl radical chosen from the group consisting of phenyl, 3-hydroxyphenyl, 3-methoxyphenyl, 2,3-dihydroxyphenyl, 2,3-dimethoxyphenyl and 1-naphthyl,
R⁹ represents H, an unsubstituted alkyl radical chosen from the group consisting of methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, or an aryl radical chosen from the group consisting of phenyl, 3-hydroxyphenyl, 3-methoxyphenyl, 2,3-dihydroxyphenyl, 2,3-dimethoxyphenyl and 1-naphthyl,
R¹¹ represents H, an unsubstituted alkyl radical chosen from the group consisting of methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, or an unsubstituted cycloalkyl radical chosen from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl; an aryl radical chosen from the group consisting of phenyl, 3-hydroxyphenyl, 3-methoxyphenyl, 2,3-dihydroxyphenyl, 2,3-dimethoxyphenyl and 1-naphthyl or an unsubstituted heteroaryl radical chosen from the group consisting of pyrrolyl, furyl, thienyl, pyrazolyl, imidazolyl, pyridinyl, pyridazinyl, pyrimidinyl, pyrazinyl, pyranyl, indolyl, indazolyl, purinyl, pyrimidinyl, indolizinyl, quinolinyl, isoquinolinyl, quinazolinyl, carbazolyl, phenazinyl and phenothiazinyl, an aryl radical which is bonded via C₁₋₃-alkylene and is chosen from the group consisting of phenyl, 3-hydroxyphenyl, 3-methoxyphenyl, 2,3-dihydroxyphenyl, 2,3-dimethoxyphenyl and 1-naphthyl or an unsubstituted heteroaryl which is bonded via C₁₋₃-alkylene and is chosen from the group consisting of pyrrolyl, furyl, thienyl, pyrazolyl, imidazolyl, pyridinyl, pyridazinyl, pyrimidinyl, pyrazinyl, pyranyl, indolyl, indazolyl, purinyl, pyrimidinyl, indolizinyl, quinolinyl, isoquinolinyl, quinazolinyl, carbazolyl, phenazinyl and phenothiazinyl, acyl C(O)R¹² or sulfonyl S(O₂)R¹³,
R¹² represents H, an unsubstituted alkyl radical chosen from the group consisting of methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, or an aryl radical chosen from the group consisting of phenyl, 3-hydroxyphenyl, 3-methoxyphenyl, 2,3-dihydroxyphenyl, 2,3-dimethoxyphenyl and 1-naphthyl,
R¹³ represents H, an unsubstituted alkyl radical chosen from the group consisting of methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, or an aryl radical chosen from the group consisting of phenyl, 3-hydroxyphenyl, 3-methoxyphenyl, 2,3-dihydroxyphenyl, 2,3-dimethoxyphenyl and 1-naphthyl,
R¹⁴ represents H, F, Br, I, Cl, OH, SH, NH₂, NO₂, CH₃, C₂H₅ , OCH₃, OC₂H₅ , OCF₃;
optionally in the form of its racemates, its pure stereoisomers, in particular enantiomers or diastereomers, or in the form of mixtures of the stereoisomers, in particular the enantiomers or diastereomers, in any desired mixture ratio;
in the form shown or in the form of its acids or its bases or in the form of its salts, in particular the physiologically acceptable salts, or in the form of its solvates, in particular the hydrates;
under the condition that the following compounds: 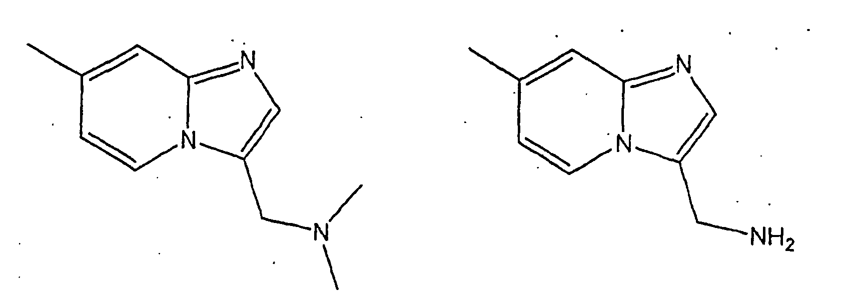 are excluded from the protection.

2. *C*-Imidazo[1,2-a]pyridin-3-yl-methylamine according to claim 1, **characterized in that** R⁴
represents a radical chosen from the group consisting of methyl, ethyl, methylethyl, 1,1-dimethylethyl, propyl, 2-methylpropyl, 1-methylpropyl, 1-ethylpropyl, butyl, i-butyl, n-butyl, tert-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl, hexyl, heptyl, octyl, nonyl, decyl, undecyl and dodecyl; in each case unsubstituted or mono- or polysubstituted by a substituent chosen from the group consisting of F, Cl, Br, I, NH₂, SH and OH; a radical chosen from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl, in each case unsubstituted or mono- or polysubstituted by a substituent chosen from the group consisting of methyl, ethyl, n-propyl,i-propyl, CF₃, methoxy and ethoxy, or a radical according to formula II 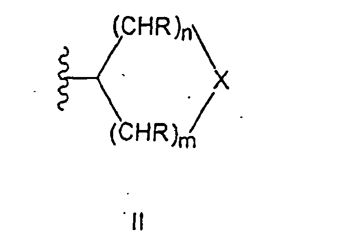 wherein in each case
n represents a number between 0 and 6,
m represents a number between 0 and 6,
1 ≤ m+n s 6,
X represents O, S, SO, SO₂ or NR⁷,
R independently of one another represent H, F, Br, I, Cl, OH, SH, NH₂, NO₂, CH₃, C₂H₅, OCH₃, OC₂H₅ , OCF₃.

3. *C*-Imidazo[1,2-a]pyridin-3-yl-methylamine according to claim 1 or 2, **characterized in that** R¹ represents C₂H₅, CH₃ or CF₃, preferably CH₃.

4. *C*-Imidazo[1,2-a]pyridin-3-yl-methylamine according to one of claims 1 to 3, **characterized in that** R⁵ represents
H; a radical chosen from the group consisting of methyl, ethyl, methylethyl, 1,1-dimethylethyl, propyl, 2-methylpropyl, 1-methylpropyl, 1-ethylpropyl, butyl, i-butyl, n-butyl, tert-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl and hexyl, in each case unsubstituted or mono- or polysubstituted by a substituent chosen from the group consisting of F, Cl, Br, I, NH₂, SH and OH.

5. *C*-Imidazo[1,2-a]pyridin-3-yl-methylamine according to one of claims 1 to 4, **characterized in that** R² represents H.

6. *C*-Imidazo[1,2-a]pyridin-3-yl-methylamine according to claim 1:
2,7-dimethyl-imidazo[1,2-a]pyridin-3-ylmethyl)-dimethyl-amine
(2,7-dimethyl-imidazo[1,2-a]pyridin-3-ylmethyl)-4-methyl-pyridin-2-yl)-amine.

7. Medicament comprising at least one *C*-imidazo[1,2-a]pyridin-3-yl-methylamine according to one of claims 1 to 6 and optionally further auxiliary substances.

8. Use of a compound according to one of claims 1 to 6 for the preparation of a medicament for the treatment of migraine, septic shock, neurodegenerative diseases, preferably multiple sclerosis, Parkinson's disease, Alzheimer's disease or Huntington's disease, inflammatory pain, cerebral ischaemia, diabetes, meningitis, arteriosclerosis or cancer diseases or for wound healing.

## Revendications

1. *C*-imidazo[1,2-a]pyridine-3-yl-méthylamine substituée de formule générale I, 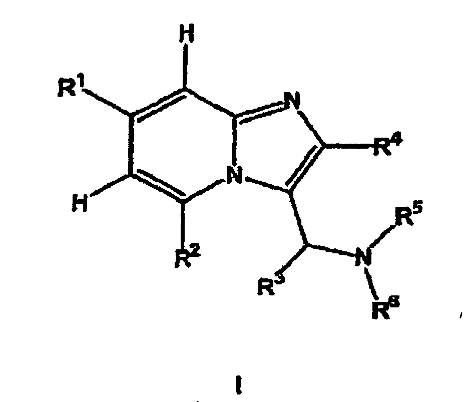 où, dans chaque cas
R¹ représente OH, SH, NH₂, NHCH₃, N(CH₃)₂, CH₃, CH₂Cl, CH₂F, CHF₂, CF₃, OCH₃, OCH₂Cl, OCH₂, OCHF₂, OCF₃, C₂H₅, CHClCH₃, CH₂CH₂Cl, CHFH₃, OH₂CH₂F, CH₂CHF₂, CH₂CF₅, OC₂H₅ COOH, CH₂OH, CNOHCH₃ ou CH₂CH₂OH
R² représente H ou CH₃,
R³ représente H,
R⁴ représente H ; un reste choisi dans le groupe constitué des restes méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-wéthylbutyle, 2-méthylbutyle, 3-méthylbutyle, 2,2-diméthylbutyle, pentyle, 2-méthcylpentyle, 3-méthylpentyle, 1,1-diméthylpropyle, 1,2-diméthylpropyle, 2,2-diméthylpropyle, 1-éthylpropryle, hexyle, heptyle, octyle, nonyle, décyle, undécyle et docécyle ; chacun non substitué ou substitué une ou plusieurs fois avec un substituant choisi dans le groupe constitué de F, Cl, Br, I, NH₂, SH et OH ; un reste choisi dans le groupe constitué des restes cyclopropyle, cyclobutyle, cyclopentyle, cyclohexyle, cycloheptyle, cyclo-octyle, cyclopentényle, cyclohexényle, cycloheptényle et cyclo- octényle, chacun non substitué ou substitué une ou plusieurs fois avec un substituant choisi dans le groupe constitué de méthyle, éthyle, n-propyle, isopropyle, CF₃, méthoxy et éthoxy ; ou bien un reste répondant à la formule II : 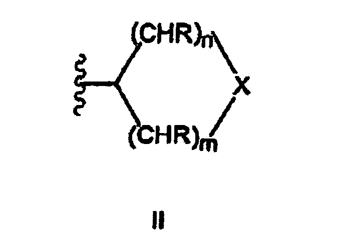
où, dans chaque cas
n est un nombre compris entre 0 et 6,
m est un nombre compris entre 0 et 6,
1 ≤ m + n ≤ 6,
X représente O, S, SO, SO₂ ou NR⁷,
R indépendamment les uns des autres, représentent H, F, Br, I, Cl, OH, SH, NH₂, NO₂, CH₃, C₂H₅, OCH₃, OCH₂H₅, OCF₃,
R⁵ représente H, un reste choisi dans le groupe constitué des restes méthyle, éthyle, méthyléthyle, 1,1-diméthyléthyle, propyle, 2-méthylpropyle, 1-méthyl-propyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthyl-pentyle, 3-méthylpentyle, 1,1-diméthylpropyle, 1,2-diméthylpropyle, 2,2-diméthylpropyle, 1-éthylpropyle et hexyle, chacun non substitué ou substitué une ou plusieurs fois avec un substituant choisi dans le groupe constitué de F, Cl, Br, I, NH₂, SH et OH ; ou bien un reste choisi dans le groupe constitué des restes cyclopropyle, cyclobutyle, cyclopentyle, cyclohexyle, cycloheptyle, cyclo-octyle, cyclopentényle, cyclohexényle, cycloheptényle et cyclo-octényle, chacun non substitué ou substitué une ou plusieurs fois avec un substituant choisi dans le groupe constitué de méthyle, éthyle, n-propyle, isopropyle, CF₃, méthoxy et éthoxy ;
R⁶ représente H ; un reste choisi dans le groupe constitué des restes méthyle, éthyle, méthyléthyle, 1,1-diméthyléthyle, propyle, 2-méthylpropyle, 1-méthylpropyle, 1-éthylpropylé, butyle, isobutyle, n-butyle, tertiobutyle, 1-méthylbutyle, 2-méthylbutyle, 3-méthylbutyle, 2,2-diméthylbutyle, pentyle, 2-méthyl-pentyle, 3-méthylpentyle, 1,1-diméthylpropyle, 1,2-diméthylpropyle, 2,2-diméthylpropyle, 1-éthylpropyle et hexyle ; chacun non substitué ou substitué une ou plusieurs fois avec un substituant choisi dans le groupe constitué de F, Cl, Br, I, NH₂, SH et OH ; un reste choisi dans le groupe constitué des restes cyclopropyle, cyclobutyle, cyclopentyle, cyclohexyle, cycloheptyle, cyclo-octyle, cyclopentényle, cyclohexényle, cycloheptényle et cyclo-octényle, chacun non substitué ou substitué une ou plusieurs fois avec un substituant choisi dans le groupe constitué de méthyle, éthyle, n-propyle, isopropyle, CF₃, méthoxy et éthoxy ; ou un reste répondant à la formule IIa : 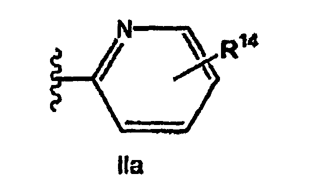
ou bien les restes R⁵ et R⁶ forment ensemble un noyau et représentent CH₂CH₂OCH₂CH₂, CH₂CH₂NR¹¹CH₂CH₂ ou (CH₂)₃₋₆,
R⁷ représente H, un reste choisi dans le groupe constitué des restes méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, 1-éthylpropyle et hexyle ; un reste choisi dans le groupe constitué des restes cyclopropyle, 2-méthylcyclopropyle, cyclopropylméthyle, cyclobutyle, 2-méthylcyclobutyle, 3-méthylcyclobutyle, cyclobutylméthyle, cyclopentyle, cyclohexyle, cyclooctyle; un reste acyle C(O)R⁸ ou un reste sulfonyle S(O₂)R⁹,
R⁸ représente H, un reste alkyle non substitué choisi dans le groupe constitué des restes méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, hexyle, 1-méthylpentyle ou un reste aryle choisi dans le groupe constitué des restes phényle, 3-hydroxyphényle, 3-méthoxyphényle, 2,3-dihydroxyphényle, 2,3-diméthoxyphényle et 1-naphtyle,
R⁹ représente H, un reste alkyle non substitué choisi dans le groupe constitué des restes méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, hexyle, 1-méthylpentyle ou un reste aryle choisi dans le groupe constitué des restes phényle, 3-hydroxyphényle, 3-méthoxyphényle, 2,3-dihydroxyphényle, 2,3-diméthoxyphényle et 1-naphtyle,
R¹¹ représente H, un reste alkyle non substitué choisi dans le groupe constitué des restes méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, hexyle, 1-méthylpentyle ou un reste cycloalkyle non substitué choisi dans le groupe constitué des restes cyclopropyle, cyclobutyle, cyclopentyle, cyclohexyle, cycloheptyle, cyclooctyle, cyclopentényle, cyclohexényle, cycloheptényle et cyclo-octényle ; un reste aryle choisi dans le groupe constitué des restes phényle, 3-hydroxyphényle, 3-méthoxyphényle, 2,3-dihydroxyphényle, 2,3-diméthoxy- phényle et 1-naphtyle, ou bien un reste hétéroaryle non substitué choisi dans le groupe constitué des restes pyrrolyle, furyle, thiényle, pyrazolyle, imidazolyle, pyridinyle, pyridazinyle, pyrimidinyle, pyrazinyle, pyrannyle, indolyle, indazolyle, purinyle, pyrimidinyle, indolizinyle, quinolinyle, isoquinolinyle, quinazolinyle, carbazolyle, phénazinyle et phénothiazinyle, un reste aryle, lié par l'intermédiaire d'un reste alkylène en C₁ à C₃, choisi dans le groupe constitué des restes phényle, 3-hydroxyphényle, 3-méthoxyphényle, 2,3-dihydroxyphényle, 2,3-diméthoxyphényle et 1-naphtyle, ou bien un reste hétéroaryle non substitué, lié par l'intermédiaire d'un reste alkylène en C₁ à C₃, choisi dans le groupe constitué des restes pyrrolyle, furyle, thiényle, pyrazolyle, imidazolyle, pyridinyle, pyridazinyle, pyrimidinyle, pyrazinyle, pyrannyle, indolyle, indazolyle, purinyle, pyrimidinyle, indolizinyle, quinolinyle, isoquinolinyle, quinazolinyle, carbazolyle, phénazinyle et phénothiazinyle, un reste acyle C(O)R¹² ou un reste sulfonyle S(O₂)R¹³,
R¹² représente H, un reste alkyle non substitué choisi dans le groupe constitué des restes méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, hexyle, 1-méthylpentyle, ou un reste aryle choisi dans le groupe constitué des restes phényle, 3-hydroxyphényle, 3-méthoxyphényle, 2,3-dihydroxyphényle, 2,3-diméthoxyphényle et 1-naphtyle,
R¹³ représente H, un reste alkyle non substitué choisi dans le groupe constitué des restes méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, hexyle, 1-méthylpentyle, ou un reste aryle choisi dans le groupe constitué des restes phényle, 3-hydroxyphényle, 3-méthoxyphényle, 2,3-dihydroxyphênyle, 2,3-diméthoxyphényle et 1-naphtyle,
R¹⁴ représente H, F, Br, I, Cl, OH, SH, NH₂, NO₂, CH₃, C₂H₅, OCH₃; OCH₂H₅, OCF₃ ;
le cas échéant sous forme de leurs racémates, de leurs stéréoisomères purs, en particulier de leurs énantiomères ou diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels acceptables du point de vue physiologique, ou sous forme de leurs produits de solvatation, en particulier des hydrates ;
sous réserve que les composés suivants : 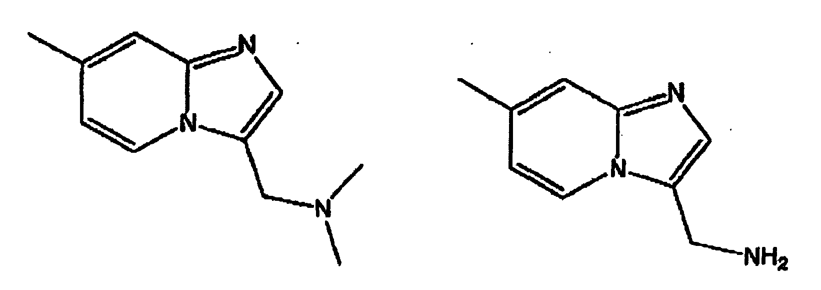 soient exclus de la protection.

2. *C*-imidazo[1,2-a]pyridine-3-yl-méthylamine suivant la revendication 1, **caractérisée en ce que** R⁴ représente
un reste choisi dans le groupe constitué des restes méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle, hexyle, heptyle, octyle, nonyle, décyle, undécyle et dodécyle ; chacun non substitué ou substitué une ou plusieurs fois avec un substituant choisi dans le groupe des substituants F, Cl, Br, I, NH₂, SH et OH ; un reste choisi dans le groupe constitué des restes cyclopropyle, cyclobutyle, cyclopentyle, cyclohexyle, cycloheptyle, cyclo-octyle, cyclopentényle, cyclohexényle, cycloheptényle et cyclo-octényle, chacun non substitué ou substitué une ou plusieurs fois avec un substituant choisi dans le groupe constitué des substituants méthyle, éthyle, n-propyle, isopropyle, CF₃, méthoxy et éthoxy, ou bien un reste de formule II : 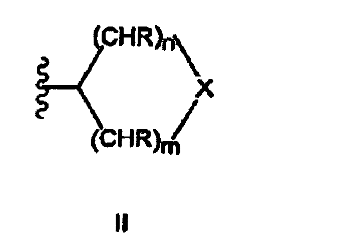 où, dans chaque cas
n est un nombre compris entre 0 et 6,
m est un nombre compris entre 0 et 6,
1 ≤ m + n ≤ 6,
X représente O, S, SO, SO₂ ou NR⁷,
R, indépendamment les uns des autres, représentent H, F, Br, I, Cl, OH, SH, NH₂, NO₂, CH₃, C₂H₅, OCH₃, OCH₂H₅, OCF₃.

3. *C*-imidazo[1,2-a]pyridine-3-yl-méthylamine suivant la revendication 1 ou 2, **caractérisée en ce que** R¹ représente C₂H₅, CH₃ ou CF₃, avantageusement CH₃.

4. *C*-imidazo[1,2-a]pyridine-3-yl-méthylamine suivant l'une des revendications 1 à 3, **caractérisée en ce que** R⁵ représente
H ; un reste choisi dans le groupe constitué des restes méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle et hexyle, chacun non substitué ou substitué une ou plusieurs fois avec un substituant choisi dans le groupe des substituants F, Cl, Br, I, NH₂, SH et OH.

5. *C*-imidazo[1,2-a]pyridine-3-yl-méthylamine suivant l'une des revendications 1 à 4, **caractérisée en ce que** R² représente H.

6. *C*-imidazo[1,2-a]pyridine-3-yl-méthylamine suivant la revendication 1 :
2,7-diméthyl-imidazo[1,2-a]pyridine-3-ylméthyl)diméthyl-amine
(2,7-diméthyl-imidazo [1,2-a] pyridine-3-ylméthyl)-4-méthyl-pyridine-2-yl)-amine.

7. Médicament contenant au moins une *C*-imidazo[1,2-a]-pyridine-3-yl-méthylamine suivant l'une des revendications 1 à 6 ainsi que, le cas échéant, d'autres substances auxiliaires.

8. Utilisation d'un composé suivant l'une des revendications 1 à 6, pour la préparation d'un médicament destiné au traitement de la migraine, du choc septique, de maladies neurodégénératives, avantageusement de la sclérose en plaques, de la maladie de Parkinson, de la maladie d'Alzheimer ou de la maladie de Huntington, d'une douleur d'origine inflammatoire, de l'ischémie cérébrale, du diabète, de la méningite et de l'artériosclérose, de maladies cancéreuses, ou pour la cicatrisation.
